(19) Europäisches Patentamt
European Patent Office
Office européen des brevets

(11) Numéro de publication: **0 002 147**
**B1**

(12) **FASCICULE DE BREVET EUROPEEN**

(21) Numéro de dépôt: **78400166.1**

(22) Date de dépôt: **08.11.78**

(51) Int. Cl³: **C 07 C 3/04**

(54) **Procédé de préparation de la tris(oxa-3 hydroxy-5 pentyl)amine**

(30) Priorité: **16.11.77 FR 7734393**

(43) Date de publication de la demande: **30.05.79 Bulletin 79/11**

(45) Mention de la délivrance du brevet: **23.07.80 Bulletin 80/15**

(84) Etats contractants désignés: **BE CH DE GB NL**

(56) Documents cités:
**US - A - 2 293 494**
**J. Am. Chem. Soc. vol. 70 (1948) pages 3098—3100**

(73) Titulaire: **Rhone-Poulenc Industries**
**22, avenue Montaigne**
**F - 75008 Paris (FR)**

(72) Inventeur: **Soula, Gérard**
**33, rue Nungesser**
**F - 69330 Meyzieu**
**France**
**Leludec, Joel**
**11, rue de Boyer**
**F - 69005 Lyon (FR)**

(74) Mandataire: **Fabre, Madeleine-France**
**Rhone Poulenc Service Brevets Chimie et Polymères B.P. 753**
**F - 75360 Paris Cedex 08 (FR)**

**EP 0 002 147 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de préparation de la tris(oxa-3 hydroxy-5 pentyl)amine

La présente invention a pour objet un procédé de préparation de la tris(oxa-3 hydroxy-5 pentyl)amine.

Il est connu d'après le brevet anglais n° 364.000 de préparer des éthers hydroxylés d'hydroxy-alkylamines tertiaires, par action d'un oxyde d'alkylène sur un composé aminé contenant au moins un atome d'hydrogène actif en présence d'un diluant aqueux.

Un tel procédé ne permet d'obtenir des éthers hydroxylés qu'avec de faibles rendements et une faible sélectivité; ainsi par action de l'oxyde d'éthylène sur l'ammoniaque, on obtient un mélange d'éthers hydroxylés, avec un rendement maximum de 20% en tris(oxa-3 hydroxy-5 pentyl)amine.

Il est connu d'après le brevet américain n° 2.293.494 de préparer des aminoéthers par action d'un alcoolate de sodium d'alcanolamine (triéthanolamine par exemple) sur une chlorhydrine (le chloro-2 éthanol par exemple). Un tel procédé ne peut être un procédé industriel car il présente l'inconvénient de devoir être réalisé avec d'extrêmes précautions en raison de la forte exothermicité de la réaction mise en jeu; ainsi il est très important de ne pas mettre en présence la totalité de l'alcoolate à faire réagir avec la totalité de la chlorhydrine nécessaire à la réaction; il est conseillé d'ajouter lentement l'alcoolate petit à petit à la chlorhydrine en agitant et en refroidissant constamment mais en maintenant toutefois une température suffisante pour que la réaction ait lieu.

La demanderesse a trouvé un procédé permettant d'obtenir d'une manière simple et industrielle de la tris(oxa-3 hydroxy-5 pentyl)-amine avec une grande sélectivité et une grande pureté.

Le procédé de préparation de tris(oxa-3 hydroxy-5 pentyl)amine objet de l'invention est caractérisé en ce que l'on condense un monoglycolate de métal alcalin en solution dans du glycol sur du chlorhydrate de tris(chloro-2 éthyl)amine selon un rapport molaire monoglycolate/chlorhydrate compris entre 4 et 5, puis en ce que l'on distille la la tris(oxa-3 hydroxy-5 pentyl)amine obtenue.

Pour une bonne réalisation de l'invention, l'opération de condensation est réalisée à la température de reflux du glycol (197°C) pendant 2 à 6 heures, à l'aide d'une solution glycolique contenant de 0,5 à 5 moles de monoglycolate de métal alcalin, de préférence de 2 à 4 moles par litre de glycol.

Parmi les solutions de monoglycolates de métaux alcalins pouvant être mises en oeuvre, on peut citer celles de sodium et de potassium.

Le schéma de la réaction de condensation, à partir de monoglycolate de sodium, par exemple, est le suivant:

$$Cl^- \; H\overset{+}{N}(CH_2-CH_2-Cl)_3 + 4\; Na^{+-}O-CH_2-CH_2-OH \rightarrow N(CH_2-CH_2-O-CH_2-CH_2-OH)_3 + 4\; ClNa$$

Les solutions glycoliques de monoglycolate de sodium ou de potassium à mettre en oeuvre, peuvent être préparées d'une manière simple et sélective, à partir de soude ou de potasse et de glycol, selon des quantités correspondant à 0,5 à 5 moles de soude ou de potasse, de préférence de 2 à 4 moles, pour un litre de glycol.

Le chlorhydrate de tris (chloro-2 éthyl)amine à mettre en oeuvre peut être préparé d'une manière connue à partir de chlorhydrate de triéthanolamine et de chlorure de thionyle, selon la méthode décrite par K. WARD dans J.A.C.S. 57, 914, 1935 ou par J. P. MASON et D. J. GASCH dans J.A.C.S. 60, 2816, 1938.

La tris (oxa-3 hydroxy-5 pentyl)amine préparée selon le procédé de l'invention peut être utilisée comme intermédiaire pour la préparation d'agents d'adoucissement pour l'industrie textile.

Les exemples suivants sont donnés à titre indicatif et ne peuvent être considérés comme une limite du domaine et de l'esprit de l'invention.

## EXEMPLE 1

*Préparation de la solution glycolique de monoglycolate de sodium*

Dans un ballon de 3 litres, muni d'un agitateur mécanique, d'un thermomètre, d'une tête de distillation suivie d'un réfrigérant et d'un ballon récepteur, on introduit 2200 g (soit 35,5 moles ou 2 litres) de glycol et 218,5 g (5,35 moles) de soude en pastilles (à 98 %).

On chauffe à 85°C, la soude se dissout totalement; on chauffe ensuite à 125°C sous 2666 Pa (20 mmHg) pour éliminer la majorité de l'eau formée (80 ml) puis on récupère 180 cm3 de glycol distillant entre 125 et 135°C. On obtient 2262 g (soit environ 2 litres) de solution glycolique contenant 2,7 moles/1 de monoglycolate de sodium; celle-ci est ensuite refroidie jusqu'à 80°C.

*Préparation de la tris(oxa-3 hydroxy-5 pentyl)amine*

Aux 2262 g de solution glycolique de monoglycolate de sodium ci-dessus préparés, (soft 5,35 moles de monoglycolate de sodium) on ajoute 280,4 g (soit 1,16 mole) de chlorhydrate de tris(chloro-2 éthyl)amine et/ou chauffe au reflux (197°C) pendant 3 heures. On observe la précipitation du chlorure de sodium formé.

Une grande partie du glycol (1450 ml) est ensuite distillée sous vide (à 100°C sous 400

Pa soit 3 mm de mercure) pendant 4 h 30. Le contenu du ballon est alors filtré, le gâteau est, lavé, par 6 fois 100 ml d'acétone puis séché.

On obtient 270 g de NaCl (au lieude 272 g selon la théorie).

Le monoglycolate de sodium en excés est neutralisé avec 0,7 mole (soit 56,5 ml) d'acide chlorhydrique (d—1,19).

L'acétone est évaporée et le résidu huileux obtenu est distillé. On recueille le coeur de distillation entre 210—220°C sous 266 Pa (2 mm de mercure).

On récupère 213 g d'un liquide jaune clair. La microanalyse et les analyses infra-rouge et R.M.N (résonnance magnétique nucléaire) confirment que ce liquide jaune clair est la tris-(hydroxy-5 oxa-3 pentyl)amine.

EXEMPLE 2

*Préparation de la solution glycolique de monoglycolate de potassium*

On introduit dans le ballon décrit à l'exemple 1, 2220 g (soit 35,5 moles) ou 2 litres de glycol et 300 g (soit 5,35 moles) de potasse en pastilles.

On chauffe le mélange à 130°C sous 2666 Pa (20 mm de mercure) pour éliminer la majorité de l'eau formée (35 ml) puis on récupère 185 cm3 de glycol. On obtient 2347 g (soit 2 litres) de solution glycolique contenant 2,7 moles/1 de monoglycolate de potassium; celle-ci est refroidie jusqu'à 80°C.

*Préparation de la tris(oxa-3 hydroxy-5 pentyl)amine*

Aux 2347 g de solution glycolique de monoglycolate de potassium ci-dessus préparés (soit 5,35 moles de monoglycolate) on adjoute 280,4 g (soit 1,16 mole) de chlorhydrate de tris(chloro-2 éthyl)amine et on chauffe au reflux pendant 3 heures. On observe la précipitation du chlorure de potassium formé.

Une grande partie du glycol (1450 ml) est ensuite distillée sous vide (à 100°C sous 400 Pa soit: 3 mm de mercure) pendant 4 h 30.

Le contenu du ballon est alors filtré, le gâteau est lavé par 6 fois 100 ml d'acétone, puis séché.

On obtient 341 g de KCl (au lieu de 346 g selon la théorie).

Le monoglycolate de potassium en excès est neutralisé avec 0,7 mole (soit 56,5 ml) d'HCl (d = 1,19), L'acétone est évaporée et le résidu huileux distillé. On recueille le coeur de distillation entre 210 et 220°C sous 266 Pa (2 mm de mercure).

On récupère 245 g de liquide jaune clair, qui est de la tris(hydroxy-5 oxa-3 pentyl)amine.

## Revendications

1. Procédé de préparation de la tris(hydroxy-5 oxa-3 pentyl)amine, caractérisé en ce que l'on condense un monoglycolate de métal alcalin en solution dans du glycol sur du chlorhydrate de tris(chloro-2 éthyl)amine selon un rapport molaire monoglycolate/chlorhydrate compris entre 4 et 5, puis en ce que l'on distille la tris-(oxa-3 hydroxy-5 pentyl)amine obtenue.

2. Procédé selon la revendication 1 caractérisé en ce que la solution de monoglycolate dans le glycol contient de 0,5 à 5 moles de monoglycolate par litre de glycol.

3. Procédé selon la revendication 2 caractérisé en ce que la solution de monoglycolate dans le glycol contient de 2 à 4 moles de monoglycolate par litre de glycol.

4. Procédé selon l'une des revendications 1, 2 ou 3 caractérisé en ce que le monoglycolate mis en oeuvre est choisi parmi le monoglycolate de sodium et le monoglycolate de potassium.

5. Procédé selon la revendication 1, caractérisé en ce que l'opération de condensation est réalisée à la température de reflux du glycol pendant 2 à 6 heures.

## Claims

1. A process for preparing tris(5-hydroxy 3-oxa pentyl)amine, characterised in that an alkali-metal monoglycolate dissolved in glycol is condensed on tris(2-chloroethyl)amine hydrochloride in a molar ratio monoglycolate/chlorhydrate between 4 and 5, and then the tris(5-hydroxy 3-oxa pentyl)amine obtained is distilled.

2. A process according to claim 1, characterised in that the solution of monoglycolate in glycol contains from 0.5 to 5 mols of monoglycolate per liter of glycol.

3. A process according to claim 2, characterised in that the solution of monoglycolate in glycol contains from 2 to 4 mols of monoglycolate per liter of glycol.

4. A process according to any of claims 1, 2 or 3 characterised in that the monoglycolate used is selected from among sodium monoglycolate and potassium monoglycolate.

5. A process according to claim 1, characterised in that the condensation operation is carried out at the reflux temperature of the glycol for about 2 to 6 hours.

## Patentansprüche

1. Verfahren zur Herstellung von Tris (5-hydroxy 3-oxa pentyl)amin, dadurch gekennzeichneit, dass man einen in Glykol gelösten Monoglykolat eines Alkalimetalls mit Tris(2-chloräthyl) amin- Chlorhydrat in einem zwischen 4 und 5 liegenden Molverhältnis Monoglykolat/Chlorhydrat kondensiert, anschliessend das erhaltene Tris(3-oxa 5-hydroxy pentyl)amin abdestilliert.

2. Verfahren nach Anspruch 1, dadurch gekennzeichnet, dass die Lösung von Monoglykolat in Glykol 0,5—5 Mol Monoglykolat je Liter Glykol enthält.

3. Verfahren nach Anspruch 2, dadurch gekennzeichnet, dass die Lösung von Monoglykolat in Glykol 2—4 Mol Monoglykolat je Liter Glykol enthält.

4. Verfahren nach einem der Ansprüche 1, 2 oder 3, dadurch gekennzeichnet, dass der eingesetzte Monoglykolat unter dem Mononatrium glykolat und dem Monokalium glykolat gewählt wird.

5. Verfahren nach Anspruch 1, dadurch gekennzeichnet, dass man 2—6 Stunden bei der Rückflusstemperatur des Glykols kondensiert.